# FASCICULE DE BREVET EUROPEEN

(11) **EP 0 951 276 B1**
(45) Date de publication et mention de la délivrance du brevet: **19.06.2002**
(21) Numéro de dépôt: 97952061.6
(22) Date de dépôt: 15.12.1997
(51) Int. Cl.: A61K 7/48

(54) **COMPOSITION COSMETIQUE SOUS FORME D'EMULSION COMPRENANT UN COLORANT POLYMERIQUE**
KOSMETISCHE ZUBEREITUNG IN FORM EINES WÄSSRIGEN GELS WELCHES EINEN POLYMERISCHEN FARBSTOFF ENTÄHLT
COSMETIC COMPOSITION IN THE FORM OF AN EMULSION CONTAINING A POLYMERIC COLOURING AGENT

(30) Priorité: 16.12.1996 FR 9615452
(43) Date de publication de la demande: 27.10.1999
(73) Titulaire: L'OREAL, 75008 Paris (FR)
(72) Inventeur: LEMANN, Patricia, F-94000 Créteil (FR); MELLUL, Myriam, F-94240 L'Hay-les-Roses (FR)
(74) Mandataire: Brédeville, Odile Marie
(86) Numéro de dépôt international: FR9702302
(87) Numéro de publication internationale: WO9826756

(56) Documents cités:
- DE-A- 1 906 841
- FR-A- 2 695 033
- US-A- 4 740 581
- US-A- 4 803 255
- US-A- 4 804 719
- US-A- 4 808 677
- US-A- 5 102 980
- US-A- 5 194 463
- DATABASE WPI Week 9616 Derwent Publications Ltd., London, GB; AN 96-157114 XP002040376 & JP 08 041 140 A (TOYOBO KK)
- DATABASE WPI Week 9435 Derwent Publications Ltd., London, GB; AN 94-282958 XP002040377 & JP 06 210 708 A (TOPPAN PRINTING CO LTD)
- DATABASE WPI Week 8937 Derwent Publications Ltd., London, GB; AN 89-266929 XP002040378 & JP 01 193 380 A (PENTEL KK)
- DATABASE WPI Week 8523 Derwent Publications Ltd., London, GB; AN 78-07550 XP002040379 & JP 52 148 632 A (POLA KASEI KOGYO KK)

## Description

L'invention a pour objet de nouvelles émulsions comprenant au moins un colorant polymérique particulier.

On utilise habituellement comme matière colorante dans les compositions cosmétiques, soit des pigments minéraux ou organiques ou des laques, qui sont généralement insolubles dans les milieux aqueux et organiques, soit des colorants solubles dans les milieux aqueux ou organiques.
Les pigments et laques utilisés dans le domaine du maquillage sont d'origine et de nature chimique très diverses. Leurs propriétés physicochimiques : granulométrie, surface spécifique, densité, etc., sont donc très différents. Ces différences se traduisent par des variations de comportement : la facilité de mise en oeuvre, de dispersion, la stabilité à la lumière, à la température et les propriétés mécaniques dans le cas des poudres sont propres à chaque matière colorante. Ces différences de comportement obligent fréquemment le formulateur à réadapter la composition de la formule lorsque la teinte de cette formule est modifiée. Elles limitent également la réalisation de certaines formules sous forme de monochromes. En particulier, certaines teintes sont irréalisables du fait que les pigments ou les laques qui permettraient théoriquement de les obtenir sont incompatibles entre eux.

On utilise de préférence les oxydes minéraux pour la coloration des compositions cosmétiques, car les laques organiques présentent un dégorgement trop important, ce qui a en particulier pour conséquence de tâcher les lentilles oculaires dans le cas des eye-liners ou des mascaras. Cependant, ces pigments minéraux sont de couleur terne et fade, il est donc nécessaire d'en introduire une grande quantité dans les formules de mascara et d'eye-liner pour obtenir un trait suffisamment saturé. Ce fort pourcentage de particules minérales affecte fortement la brillance du film de maquillage. Dans le cas des rouges à lèvres, le problème est identique, les compositions brillantes doivent être formulées avec des petites quantités de pigments. On associe donc souvent couvrance et saturation de la couleur, à la matité du film de maquillage.

Dans le domaine du maquillage, seules les laques organiques permettaient jusqu'à présent d'obtenir des couleurs vives et intenses (les oxydes minéraux ont une couleur très fade). Cependant, la plupart des laques organiques présentent une très mauvaise tenue à la lumière, qui se traduit par une atténuation très nette de leur couleur dans le temps. Ce phénomène est encore aggravé lorsque ces laques sont associées à des pigments photoréactifs comme le dioxyde de titane. Or ces pigments photo-réactifs sont très largement utilisés dans le maquillage pour la protection contre les rayonnement ultra-violets. Par conséquent, l'utilisation des laques organiques est assez limitée, ce qui a pour conséquence une limitation des teintes réalisables.

L'association de certains pigments, en particulier des oxydes de fer, avec des huiles et certains actifs, dont notamment les vitamines, a souvent pour conséquence l'oxydation de ces huiles et de ces actifs.

Lorsque l'on prépare une émulsion eau dans huile ou huile dans eau, dans laquelle la phase grasse comporte une huile de silicone, on est habituellement contraint d'utiliser des pigments enrobés, coûteux, car tout autre type de pigment déstabilise l'émulsion.

Dans les compositions de mascara on utilise jusqu'à 10 % de pigments d'oxydes minéraux pour obtenir une coloration visible et acceptable sur les cils. Cependant, une aussi forte concentration de pigments entraîne une forte diminution des performances rhéologiques du mascara, et produit donc un maquillage hétérogène sur les cils. Les laques organiques, qui permettent l'obtention de couleurs beaucoup plus vives à des pourcentages inférieurs sont peu utilisables pour cette application car comme on l'a déjà indiqué, elles dégorgent facilement et donc, risquent de tâcher les lentilles.

En outre, l'utilisation de colorants solubles dans des compositions de maquillage a pour effet d'accentuer les rides ou stries de la peau, le colorant venant se fixer, par migration, préférentiellement dans ces rides ou stries. Cet effet est contraire à celui d'atténuation des défauts de la peau que l'on cherche à obtenir par le maquillage. Ces colorants solubles présentent souvent l'inconvénient de laisser après le démaquillage des taches sur la peau et les ongles, avec lesquels ils ont une grande affinité.

Enfin, si les pigments minéraux doivent être utilisés en grandes quantités pour obtenir une coloration satisfaisante, en revanche, les colorants solubles ont un pouvoir colorant très élevé, ce qui conduit à les utiliser en quantités infinitésimales. Par conséquent leur dosage dans une formule cosmétique dans des conditions reproductibles est une opération extrêmement délicate.

Il subsistait donc le besoin de disposer de matière colorante nouvelle permettant de pallier les inconvénients de l'art antérieur.

Aussi c'est avec étonnement que la demanderesse a découvert que l'utilisation d'un colorant polymérique appartenant à une famille particulière, pouvait permettre de préparer des compositions cosmétiques nouvelles, qui ne présentent plus les inconvénients de l'art antérieur.

En particulier, ces compositions ne relarguent pas de colorant sur la peau, ce qui évite l'apparition de stries sur la peau maquillée ou de tâches après le démaquillage. Elles permettent ainsi une coloration non permanente de la peau, des ongles, des lèvres, ou des cils : après le démaquillage, la peau, les ongles, les lèvres ou les cils ne sont pas colorés. On obtient ainsi un démaquillage net. On dit que les compositions "ne griffent pas".
Ces compositions sont préparées à partir de quantités de colorant polymérique suffisamment faibles pour ne pas déstabiliser les formules, mais en quantités suffisamment importantes pour être facilement dosées de façon reproductible.

En outre, ces compositions peuvent être préparées suivant des formules invariables quelle que soit la teinte souhaitée, avec une gamme de teintes très large et très nuancée, avec une grande intensité de coloration associée à un effet brillant si cela est souhaité, avec une bonne tenue à la lumière, y compris lorsque la composition comprend, en outre, des pigments photoréactifs.

L'invention a pour objet une émulsion comprenant de l'eau, un composant gras choisi parmi les huiles éventuellement volatiles et/ou les cires et un colorant polymérique caractérisée en ce que le colorant polymérique est choisi parmi les polymères sulfopolyester, polyamide, polyuréthanne ou leurs mélanges.

Par colorant polymérique, on entend dans la présente description, un copolymère à base d'au moins deux monomères distincts dont l'un au moins est un monomère colorant organique.

De tels colorants polymériques sont connus de l'homme du métier. On peut, par exemple, se référer aux documents : US-5,194,463 ; US-4,804,719.
Il est exposé dans ces documents que ces colorants ont la propriété de ne pas migrer, de ne pas exsuder, de ne pas être extractibles ou sublimables. Ils sont également réputés être stables à la lumière et présenter un fort pouvoir colorant. Toutefois, les milieux dans lesquels leurs mise en oeuvre est envisagée dans ces documents sont des milieux thermoplastiques, essentiellement pour la fabrication d'emballages. Rien ne laisse prévoir les propriétés étonnantes de ces polymères une fois qu'ils sont incorporés dans les compositions cosmétiques conformes à la présente invention.

US 5, 043, 376 décrit des colorants polyesters pouvant être broyés dans une huile minérale en vue de préparer une crème nettoyante. Toutefois, nulle part dans US 5, 043, 376 il n'est dit ou suggéré que l'incorporation d'un colorant sulfopolyester dans une émulsion comme dans la présente invention permet d'obtenir des émulsions qui ne relarguent pas de colorant sur la peau et donc qui ne tâchent pas la peau après démaquillage comme expliqué ci-dessus.

Les colorants polymériques utilisables dans la présente invention sont choisis parmi les polymères sulfopolyester, polyamide ou polyuréthanne, de préférence parmi les polymères sulfopolyester ou polyuréthanne. Ces colorants polymériques sulfopolyester ou polyuréthanne peuvent être de type cristallin, semi-cristallin ou amorphe.

Les colorants polymériques sulfopolyester utilisables selon la présente invention résultent de préférence de la polymérisation de plusieurs monomères dont :
(i) au moins un résidu acide di-carboxylique portant au moins un groupement sulfonique;
(ii) au moins un résidu diol ; et
(iii) au moins un monomère colorant.

Les polymères polyuréthanne utilisables selon la présente invention ont une viscosité intrinsèque d'au moins 0,20 et peuvent résulter de la polymérisation de plusieurs monomères dont :
(i) au moins un résidu di-isocyanate ;
(ii) au moins un résidu diol ; et
(iii) au moins un monomère colorant.

Les résidus acide dicarboxylique porteurs de groupement sulfonique peuvent être choisis parmi les diacides cycloaliphatiques comme le sulfo diacide 1,4-cyclohexane carboxylique, ou bien encore parmi les diacides aromatiques tels que les acides sulfophtaliques, l'acide 4-sulfonaphtalène-2,7-dicarboxylique.

Les résidus di-isocyanate peuvent être de type aliphatique, alicyclique ou aromatique, comme par exemple le 2,4-tolylène di-isocyanate, le 2,6-tolylène di-isocyanate, le 4,4'-biphénylène di-isocyanate, le p-xylène di-isocyanate, le méthylène di-p-phényl di-isocyanate, le p-phénylène di-isocyanate, le m-phénylène di-isocyanate, l'hexaméthylène di-isocyanate, l'isophorone di-isocyanate, etc.

Les résidus diols peuvent être choisi par exemple parmi l'éthylène glycol, le 1,2-propane diol, le 1,3-propane diol, le 2-méthyl-1,3-propanediol, le 1,4-butane diol, le 2,2-diméthyl-1,3-propanediol, le 1,6-hexanediol, le 1,10-décane diol, le 1,12-dodécane diol, le 1,2-cyclohexane diol, le 1,4-cyclohexanediol, le 1,2-cyclohexane diméthanol, le x,8-bis(hydroxyméthyl)-tricyclo-[5.2.1.0]décane, dans lequel x représente 3, 4 ou 5 ; les diols comprenant au moins un atome d'oxygène dans la chaîne comme par exemple le diéthylène glycol, le triéthylène glycol, le dipropylène glycol, le 1,3-bis (2-hydroxyéthyl) benzène, le 1,4-bis (2-hydroxyéthyl)benzène, etc.
En règle générale, ces diols comprennent 2 à 18 et préférentiellement 2 à 12 atomes de carbone.

En outre, les colorants sulfopolyester peuvent comporter des résidus acide di-carboxylique exempts de groupement sulfonique ; de tels résidus peuvent être de type aliphatique, alicyclique ou aromatique, comme par exemple les acides téréphtalique, isophtalique, 1,4-cyclohexane dicarboxylique, 1,3-cyclohexane di-carboxylique, succinique, glutarique, adipique, sébacique, 1, 2-dodécanedioïque, 2,6-naphtalène dicarboxylique, etc.

De préférence, le polymère sulfopolyester est un matériau polymère dispersible dans l'eau ayant des groupes de liaison comprenant au moins environ 20 % en moles de carbonyloxy et jusqu'à environ 80 % en moles de carbonylamido, ledit matériau contenant des groupes sulfonate hydrosolubilisants et ayant d'environ 0,01 à environ 40 % en moles, sur la base du total de tous les équivalents d'hydroxy, de carboxy ou d'amino réactifs, de colorant comprenant un ou plusieurs composés organiques thermostables ayant initialement au moins un groupe condensable, que l'on a fait réagir sur ou dans le tronc polymère. Les équivalents susmentionnés englobent leurs divers dérivés condensables, y compris des carbalcoxy, carbaryloxy, N-alkylcarbamyloxy, acyloxy, chlorocarbonyle, carbamyloxy, N-(alkyl)₂ carbamyloxy, alkylamino, N-phénylcarbamyloxy, cyclohexanoyloxy et carbocyclohexyloxy.

Dans une forme de réalisation hautement préférée de la présente invention, le matériau polymère contient des groupes de liaison carbonyloxy dans la structure moléculaire linéaire, où jusqu'à 80 % desdits groupes de liaison peuvent être des groupes de liaison carbonylamido, le polymère ayant une viscosité inhérente d'environ 0,1 à environ 1, mesurée dans une solution à 60-40 parties en poids de phénol/tétrachloroéthane, à 25 °C et à une concentration de 0,25 gramme de polymère dans 100 ml du solvant, le polymère contenant des proportions essentiellement équimolaires d'équivalents d'acide (100 pour cent en moles) par rapport aux équivalents d'hydroxy et d'amino (100 pour cent en moles), le polymère comprenant les résidus de réaction des réactifs (a), (b), (c), (d) et (e) suivants ou de leurs dérivés générateurs d'ester ou générateurs d'estéramide :
(a) au moins un acide dicarboxylique difonctionnel ;
(b) d'environ 4 à environ 25 pour cent en moles, sur la base d'un total de tous les équivalents d'acide, d'hydroxyle et d'amino étant égal à 200 pour cent en moles, d'au moins 1 sulfomonomère difonctionnel contenant au moins un groupe sulfonate cationique relié à un noyau aromatique ou cycloaliphatique où les groupes fonctionnels sont des hydroxy, carboxyle ou amino ;
(c) au moins un réactif difonctionnel choisi parmi un glycol ou un mélange d'un glycol et d'une diamine ayant deux groupes -NRH, le glycol contenant deux groupes -CH₂-OH dont
   (1) au moins 10 pour cent en moles, sur la base du pourcentage total en moles d'équivalents d'hydroxy ou d'hydroxy et d'amino, sont un polyéthylèneglycol de formule structurale :

      H-(OCH₂-CH₂)ₙ-OH

      n étant un entier de 2 à environ 20 ou
   (2) d'environ 0,1 à moins d'environ 15 pour cent en moles, sur la base du pourcentage total en moles d'équivalents d'hydroxy ou d'hydroxy et d'amino, sont un polyéthylèneglycol de formule structurale :

      H-(OCH₂-CH₂)ₙ-OH

      n étant un entier compris entre 2 et environ 500, et à condition que le pourcentage en moles dudit polyéthylèneglycol dans ladite gamme soit inversement proportionnel à la valeur de n dans ladite gamme ;
(d) de zéro à au moins un réactif difonctionnel choisi parmi un acide hydroxy-carboxylique ayant un groupe -C(R)₂-OH, un acide aminocarboxylique ayant un groupe -NRH, et un amino-alcool ayant un groupe - C(R)₂-OH et un groupe -NRH, ou des mélanges desdits réactifs difonctionnels ; où chaque R dans les réactifs (c) ou (d) est un atome H ou un groupe alkyle ayant de 1 à 4 atomes de carbone ; et
(e) d'environ 0,1 % en moles à environ 15 % en moles, sur la base d'un total de tous les équivalents d'acide, d'hydroxyle et d'amino étant égal à 200 % en moles, de monomère colorant ayant au moins un groupe acide, hydroxy ou amino que l'on a fait réagir sur ou dans la chaîne polymère.

Avantageusement, le sulfopolyester hydrodispersible comprend :
(a) un monomère acide comprenant de 75 mole % à 84 mole % d'acide isophtalique et de 25 mole % à 16 mole % de sel de sodium de l'acide sulfo-5-isophtalique,
(b) un monomère glycol comprenant de 45 à 60 mole % de diéthylène glycol et de 55 à 40 mole % de 1,4-cyclohexanediméthanol ou d'éthylèneglycol ou leur mélange,
(c) de 0,5 à 10 mole % de monomère colorant.

En particulier, les colorants polymériques polyuréthanne utilisables selon la présente invention peuvent être ceux décrits dans le brevet US-A-5194463 et répondant à la formule (I) suivante : 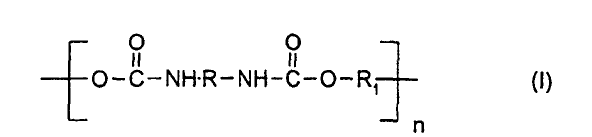 dans laquelle :
R est un radical divalent choisi parmi les radicaux alkylène en C2-C10, cycloalkylène en C3-C8, arylène, alkylène(C1-C4)-arylène-alkylène(C1-C4), alkylène(C1-C4)-cycloalkylène(C3-C8)-alkylène(C1-C4), alkylène(C1-C4)-1,2,3,4, 5,6,7-octahydronaphtalène-2,6-diyl-alkylène(C1-C4),
R¹ est un radical divalent organique comprenant (a) de 1 à 100 moles % de diol organique colorant dans lequel les groupes hydroxyle dudit diol sont liés par un motif alkylène au reste du composé colorant, et (b) de 0 à 99 moles % de diols organiques de formule HO-R²-OH, dans laquelle R² est un radical divalent choisi parmi les radicaux alkylène en C2-C18, cycloalkylène en C3-C8, alkylène(C1-C4)-arylène-alkylène(C1-C4), alkylène(C1-C4)-cycloalkylène(C3-C8)-alkylène (C1-C4), alkylène(C1-C4)-1,2,3,4,5,6,7-octahydronaphtalène-2,6-diyl-alkylène (C1-C4), alkylène(C2-C4)-O-alkylène(C2-C4), alkylène(C2-C4)-S-alkylène(C2-C4), alkylène(C2-C4)-O-alkylène(C2-C4)-O-alkylène (C2-C4),
et n est égal ou supérieur à 2.

De préférence, R1 comprend de 5 à 50 moles pourcent de diol colorant, et n va de 2 à 100.

Pour les polymères polyuréthanne, les monomères diols colorants sont choisis parmi une variété de classes de chromophores. Ces classes de chromophores peuvent être choisis parmi les anthraquinones, les méthines, bis-méthines, les azo-méthines, les arylidènes, les 3H-dibenzo[7,i-j] isoquinolines, les phtaloylphénoxazines, les phtaloylacridone, les anthrapyrimidines, les anthrapyrazoles, les phtalocyanines, les quinophtalones, les indophénols, perinones, les nitroarylamines, benzodifurane, les 2 H-1-benzopyran-2-one, les quinophtalones, les triphénodioxazines, les fluoridines, les benzanthrones, les indigo, thioindigo, xanthène, acridine, azine, oxazine, etc, Ces monomères colorants doivent porter au moins deux groupements hydroxyles.
On pourra se référer aux brevets US-5,194,463 pour trouver des exemples de monomères colorants.

Les monomères colorants organiques selon l'invention doivent comporter au moins deux substituants susceptibles de réagir avec au moins l'un des autres monomères employés pour la préparation du colorant polymérique et doivent être stables à la température et dans les conditions de préparation dudit polymère.

Hormis ces deux conditions préliminaires, la nature chimique des monomères colorants n'a pas d'importance pour la réalisation de l'invention. Ceux-ci peuvent par exemple, être choisis parmi les anthraquinones, les méthines, bis-méthines, les aza-méthines, les arylidènes, les 3H-dibenzo[7,i-j] isoquinolines, les acides 2,5-diarylaminotéréphtaliques et leurs esters, les phtaloylphénothiazines, les phtaloylphénoxazines, les phtaloylacridone, les anthrapyrimidines, les anthrapyrazoles, les phtalocyanines, les quinophtalones, les indophénols, perinones, les nitroarylamines, benzodifurane, les 2 H-1-benzopyran-2-one, les quinophtalones, les perylènes, les quinacridones, les triphénodioxazines, les fluoridines, les 4-amino-1,8-naphtalimides, les thioxanthrones, les benzanthrones, les indanthrones, les indigo, thioindigo, xanthène, acridine, azine, oxazine, etc.
On pourra se référer aux brevets US-4,267,306 ; US-4,359,570 ; US-4,403,092 ; US-4,617,373 ; US-4,080,355 ; US-4,740,581 ; US-4,116,923 ; US-4,745,173 ; US-4,804,719, US-5,194,463 ; US-5,804,719 ; WO92/07913 pour trouver des exemples de monomères colorants utilisables dans la préparation des colorants polymériques.

Les substituants portés par le monomère colorant et susceptibles de réagir avec les autres monomères peuvent par exemple être choisis parmi les groupements suivants:
- hydroxy,
- carboxy, ester, amino, alkylamino : 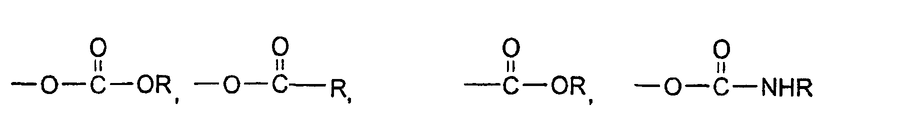
dans lesquels R représente un groupement choisi parmi les alkyles, les aryles. De préférence R est choisi parmi les groupements alkyles en C1-C8 et le phényle. Encore plus préférentiellement R est choisi parmi les groupements méthyle, éthyle et phényle.

Habituellement, le colorant polymérique comprend au moins 5 % en poids de monomère colorant, et n'en comprend pas plus de 55% en poids.
De préférence, le pourcentage en poids de monomère colorant par rapport au poids total du copolymère va de 10 à 40 %.
Pour la préparation des colorants polymériques, on peut se reporter aux procédés décrits dans les documents : US-5,194,463; US-4,804,719.

Le colorant polymérique utilisable dans la présente invention peut se présenter sous forme brute ou sous la forme d'une poudre dispersable, d'une solution en milieu aqueux, d'une dispersion en milieu aqueux ou d'une dispersion en milieu huileux.

Une dispersion en milieu aqueux d'un colorant polymérique insoluble dans l'eau peut être préparée de façon connue à partir d'eau, de colorant polymérique brut tel que décrit dans les documents US-5,032,670 ; US-4,999,418 ; US-5,106,942 ; US-5,030,708 ; US-5,102,980 ; US-5,043,376, US-5,194,463.
Par exemple on peut partir du colorant polymérique sous forme de poudre ou de granulé, et d'au moins un tensio-actif ionique, de préférence un tensio-actif anionique ou amphotère. Les tensio-actifs anioniques et amphotères peuvent être choisis préférentiellement parmi les sels alcalins d'acides gras en C12-C24, les phosphatides de soja, les phospholipides, les lysophospholipides. Ces tensio-actifs ioniques sont introduits en quantités préférentiellement comprises entre 0,5 et 30%, et encore plus préférentiellement entre 1 et 10% en poids par rapport au poids du colorant polymérique. De façon préférentielle, ces dispersions comprennent en outre au moins un tensioactif non-ionique, que l'on choisit avantageusement parmi les dérivés polyoxyéthylénés ayant un poids moléculaire supérieur à 300, préférablement aux alentours de 1000 à 15000 et une balance hydrophile-lipophile (ou balance HLB) supérieure ou égale à 10.

Par exemple, on peut préparer une dispersion aqueuse de colorant polymérique en suivant les étapes suivantes :
(i) préparation d'une émulsion huile dans eau à partir d'eau, d'une solution du colorant polymérique dans un solvant organique volatile dans lequel il est soluble, en présence d'au moins un tensio-actif ionique et éventuellement d'un tensio-actif non-ionique tels que décrits ci-dessus ;
(ii) évaporation du solvant organique volatile.

Le solvant organique volatile utilisable pour la mise en oeuvre de ce procédé doit être non miscible à l'eau et susceptible de solubiliser le polymère. De préférence son point d'ébullition est inférieur à 100°C. Il peut par exemple être choisi parmi : les solvants hydrocarbonés comme le n-hexane, le cyclohexane, le cyclopentane ; les solvants chlorés comme le chlorure de méthylène, le chloroforme ; les alkyl esters d'acides carboxyliques, comme l'acétate d'éthyle ; les dialkyléthers comme le diisopropyléther. On utilise préférentiellement pour la mise en oeuvre de ce procédé un mélange de solvants comprenant, outre les solvants décrits ci-dessus un solvant volatile, polaire, miscible à l'eau, comme l'acétone ou un alcanol de faible poids moléculaire.

Pour plus d'information sur un tel procédé, on peut se référer aux brevets US-5,043,376 et US-5,104,913.

L'eau des dispersions aqueuses décrites ci-dessus peut ensuite être évaporée, par exemple par atomisation ou par lyophilisation, afin d'obtenir une poudre d'une composition de colorant polymérique dispersable dans d'autres milieux, en particulier dans les milieux organiques tels que les huiles et les cires pour conduire à des dispersions grasses.

En employant des méthodes connues de l'homme de métier, on prépare une émulsion eau-dans-huile, huile-dans-eau, eau-dans-cire, cire-dans-eau, des émulsions triples, à partir d'eau, d'huile et/ou de cire et d'au moins un colorant polymérique tel que décrit ci-dessus. Comme il vient d'être exposé, ce colorant polymérique peut être incorporé sous forme de solution aqueuse, de dispersion aqueuse ou de dispersion huileuse, ou tel quel, sous forme de poudre dispersable ou de poudre brute. Il peut être introduit dans l'une des phases de l'émulsion avant formation de l'émulsion ou dans l'émulsion déjà formée.

Les colorants polymériques utilisables dans les compositions selon l'invention peuvent être introduits en quantités allant de 0,1 à 20% en poids.

Les colorants polymériques décrits ci-dessus ont l'avantage de présenter la même structure chimique générale et les mêmes propriétés physico-chimiques, dans des gammes de couleurs très différentes. Ainsi, un pigment rouge aura le même comportement qu'un pigment de couleur jaune. Cette homogénéité de comportement permet la fabrication des compositions de maquillages sous forme de monochromes. Lors du mélange des monochromes pour la réalisation d'une teinte, on n'observe aucun problème d'incompatibilité entre les différentes couleurs.

Ces colorants ont un pouvoir colorant supérieur aux oxydes minéraux et proche de celui des laques organiques : 4 à 5 % suffisent à donner un mascara de couleur vive et intense. Les problèmes de rhéologie sont donc fortement atténués. De plus, ces polymères colorés ne relarguent pas de colorant. Il n'y a donc aucun problème d'apparition de stries, de tâchage de la peau ou des lentilles oculaires.

Ces colorants polymériques, utilisés dans les émulsions selon l'invention ont un pouvoir colorant très proche de celui des laques organiques, et présentent une très grande stabilité à la lumière, ceci même en présence de dioxyde de titane ou de zinc. On peut donc les utiliser à la place des laques organiques dans toutes les formules de maquillage. Leur grande stabilité à la lumière permet de présenter les compositions cosmétiques les comprenant dans des emballages transparents. Ainsi, l'utilisatrice peut choisir avec plus de précision la couleur du produit qu'elle achète. En outre, ils n'interfèrent pas avec les huiles et/ou les actifs

Ces colorants polymériques de couleurs vives et intenses, ont la particularité d'être suffisamment couvrants pour qu'une petite quantité suffise à colorer une formule cosmétique. La brillance du film est donc nettement améliorée. On obtient un maquillage à la fois couvrant, saturé et brillant.

Les émulsions à base d'huile de silicone ne sont pas déstabilisées par l'introduction de pigments polymériques.

Les émulsions selon l'invention comprennent au moins une phase aqueuse, une phase grasse et un colorant polymérique selon l'invention. Elle comprennent en outre préférentiellement au moins un émulsionnant. Cet émulsionnant peut être un tensio-actif choisi parmi les tensio-actifs non-ioniques, cationiques, anioniques ou amphotères. Outre les tensio-actifs décrits ci-dessus pour la préparation des dispersions aqueuses de colorant polymérique, on peut citer comme exemple de tensioactifs utilisables dans l'invention : les émulsionnants à base de silicone et les émulsionnants lipidiques comme les alcools gras, les esters de glycérol, les esters de sorbitan, les esters de méthylglycoside et les esters de saccharose.

La phase grasse comprend au moins une huile éventuellement volatile et/ou une cire.

Selon l'invention, la cire peut être une cire animale, végétale, minérale ou synthétique. Parmi les cires animales, on peut notamment citer les cires d'abeilles, la cire de baleine. Parmi les cires végétales, on peut citer, entre autres, les cires de Carnauba, de Candellila, d'Ouricoury, les cires de fibres de liège, les cires de canne à sucre et les cires du Japon. Parmi les cires minérales, on peut citer, en particulier, les cires de paraffine, la lanoline, les cires microcristallines, les cires de lignite et les ozokérites. Parmi les cires synthétiques, on peut citer, notamment, les cires de polyéthylène et les cires obtenues par synthèse de Fisher et Tropsch. Toutes ces cires sont bien connues de l'Homme de l'Art.

On peut citer parmi les huiles utilisables dans la présente invention, l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide des huiles d'hydrocarbures telles que des huiles de paraffine, le squalane, la vaseline; des esters tels que le myristate d'isopropyle, le palmitate d'isopropyle, le stéarate de butyle, le laurate d'hexyle, l'isononanoate d'isononyle, le palmitate de 2-éthyl hexyle, le laurate de 2-hexyl décyle, le palmitate de 2-octyl décyle, le myristate de 2-octyl dodécyle, le succinate de 2-diéthylhexyle, le malate de diisostéaryle, le lactate de 2-octyl dodécyle, le triisostéarate de glycérine, le triisostéarate de glycérine, etc.; des huiles de silicone comme les polyméthylsiloxanes, les polyméthylphénylsiloxanes, des polysiloxanes modifiés par des acides gras, des polysiloxanes modifiés par des alcools gras, des polysiloxanes modifiés par des polyoxyalkylènes, des silicones fluorées, etc.; des huiles perfluorées et/ou organo-fluorées ;des acides gras supérieurs tels que l'acide myristique, l'acide palmitique, l'acide stéarique, l'acide béhénique, l'acide oléique, l'acide linoléique, l'acide linolénique, l'acide isostéarique, des alcools gras supérieurs tels que le cétanol, l'alcool stéarylique, l'alcool oléique etc.;

La phase grasse peut contenir en outre des huiles volatiles, qui s'évaporeront au contact de la peau, mais dont la présence dans la composition cosmétique est utile car elles facilitent l'étalement de la composition lors de l'application sur la peau. De tels agents d'étalement appelés ici huiles volatiles sont généralement des huiles ayant à 25°C une tension de vapeur saturante au moins égale à 0,5 millibar (soit 0,5.10²Pa).

Les huiles volatiles lorsqu'elles sont présentes représentent généralement moins de 10% en poids de la composition finale et moins de 20% en poids du liant gras. Parmi les huiles volatiles utilisables dans la présente invention on citera par exemple des huiles de silicone telles que l'hexaméthyldisiloxane, le cyclopentadiméthylsiloxane, le cyclotétraméthylsiloxane, des huiles fluorées comme celles commercialisées sous la dénomination Galden (Montefluos) ou des huiles isoparaffiniques telles que celles qui sont commercialisées sous la dénomination ISOPAR (E, G, L ou H).

Les compositions selon l'invention peuvent également comprendre les ingrédients habituellement utilisés en cosmétique parmi lesquels on peut citer les parfums, des adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les matières colorantes, les charges. Les quantités de ces différents adjuvants sont celles classiquement utilisées dans les domaines considérés, et par exemple de 0,01 % à 20 % du poids total de la composition. Ces adjuvants, selon leur nature, peuvent être introduits dans la phase grasse, dans la phase aqueuse et/ou dans les sphérules lipidiques.

Comme solvants utilisables dans l'invention, on peut citer les alcools inférieurs, notamment l'éthanol et l'isopropanol, le propylène glycol.
Comme gélifiants hydrophiles, on peut citer les polymères carboxyvinyliques (Carbomer), les copolymères acryliques tels que les copolymères d'acrylates/alkylacrylates, les polyacrylamides, les polysaccharides tels que l'hydroxypropylcellulose, les gommes naturelles (xanthane) et les argiles, et, comme gélifiants lipophiles, on peut citer les argiles modifiées comme les bentones, les sels métalliques d'acides gras comme les stéarates d'aluminium, la silice hydrophobe, les polyéthylènes et l'éthylcellulose.
Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon, les extraits bactériens ou végétaux, notamment d'Aloe Vera.
Comme actifs lipophiles, on peut utiliser le tocophérol (vitamine E) et ses dérivés, les acides gras essentiels, les céramides, les huiles essentielles.
On peut, entre autre, introduire dans les compositions selon l'invention des agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que la vitamine D et ses dérivés, les oestrogènes tels que l'oestradiol, l'acide kojique ou l'hydroquinone ;
- les agents anti-radicaux libres, tels que l'alpha-tocophérol ou ses esters, les superoxyde dismutases, certains chélatants de métaux ou l'acide ascorbique et ses esters.
Comme charges utilisables dans la présente invention, on peut citer notamment, le talc, la silice, le mica, le nitrure de bore, et comme exemples de charges organiques la poudre de Nylon, la poudre de silicone et la poudre de polyméthylène méthacrylate.

Ces compositions constituent notamment des crèmes teintées transparentes, des fonds de teint, des rouges à lèvres, des eye-liners, des mascaras, des produits de soin pour les lèvres, des poudres coulées, des fards à paupières.

De préférence, l'émulsion selon l'invention est une émulsion de maquillage.

La présente invention a encore pour objet l'utilisation d'un colorant polymérique tel que défini ci-dessus dans une émulsion pour former un film coloré sur la peau, les cils, les ongles ou les lèvres.

La présente invention a également pour objet l'utilisation d'un colorant polymérique tel que défini ci-dessus dans une émulsion qui ne colore pas la peau, les cils, les ongles ou les lèvres après le démaquillage.

La présente invention a enfin pour objet un procédé de maquillage des yeux, des cils, des ongles ou des lèvres, caractérisé par le fait que l'on applique sur les ongles, les cils, la peau ou les lèvres une émulsion telle que définie ci-dessus.

### EXEMPLES

### Exemple 1 : composition de mascara

| Phase A : | |
|---|---|
| acide stéarique | 6% |
| stéarate de glycéryle | 3,7% |
| mélange de cires | 16,7% |
| conservateur | 0,07% |

| Phase C₁ : | |
|---|---|
| hydroxyéthylcellulose | 0,2% |
| eau | 38,7% |
| conservateur | 0,2% |

| Phase C₂: | |
|---|---|
| triéthanolamine | 3% |

| Phase D: | |
|---|---|
| colorant polymérique bleu (*) | 10% |

| Phase E: | |
|---|---|
| éthoxydiglycol | 0,2% |
| conservateur | 0,03% |
| acacia | 5,8% |
| eau | qsp 100 |

| | |
|---|---|
| (*) sulfopolyester colorant comprenant 10% de monomère colorant bleu, préparé conformément à l'exemple 4 du brevet US-4,804,719 et mis en solution aqueuse à 21,6% de matière active. | |

### Mode opératoire :

On prépare C₁ sous agitation, à chaud (environ 80°C) et on laisse reposer pendant 12 heures. On prépare la phase E en évitant de chauffer. On chauffe A à 120°C puis on l'homogénéise par passage au Moritz. On chauffe C₁+C₂+D et E à 80°C et on émulsionne avec A. On laisse homogénéiser au Moritz jusqu'à ce que le mélange prenne en masse. On refroidit sous agitation lente.

### Exemple 2 : composition de mascara

| Phase A : | |
|---|---|
| acide stéarique | 6% |
| stéarate de glycéryle | 3,7% |
| mélange de cires | 16,7% |
| conservateur | 0,07% |

| Phase B : | |
|---|---|
| colorant polymérique bleu (*) | 4% |
| ultramarines et silice | 0,9% |
| dioxyde de titane | 0,5% |

| Phase C: | |
|---|---|
| hydroxyéthylcellulose | 0,2% |
| eau | 38,7% |
| conservateur | 0,2% |

| Phase D: | |
|---|---|
| triéthanolamine | 3% |

| Phase E: | |
|---|---|
| éthoxydiglycol | 0,2% |
| conservateur | 0,03% |
| acacia | 5,8% |
| eau | qsp 100 |

| | |
|---|---|
| (*) poudre de polyuréthanne comprenant 30% de monomère colorant bleu préparé conformément à l'exemple 2 du brevet US-5,194,463. | |

### Mode opératoire :

On prépare C sous agitation, à chaud (environ 80°C) et on laisse reposer pendant 12 heures. On prépare la phase E en évitant de chauffer. On chauffe A à 120°C puis on l'homogénéise par passage au Moritz. On ajoute B dans A et on poursuit l'agitation pendant 10mn. On chauffe C+D et E à 80°C et on émulsionne avec A+B. On laisse homogénéiser au Moritz jusqu'à ce que le mélange prenne en masse. On refroidit sous agitation lente.

### Exemple 3 (comparatif) : composition de mascara

| Phase A : | |
|---|---|
| acide stéarique | 6% |
| stéarate de glycéryle | 3,7% |
| mélange de cires | 16,7% |
| conservateur | 0,07% |

| Phase B: | |
|---|---|
| bleu d'outremer | 6,1% |
| ultramarines et silice | 0,9% |
| dioxyde de titane | 0,5% |

| Phase C: | |
|---|---|
| hydroxyéthylcellulose | 0,2% |
| eau | 38,7% |
| conservateur | 0,2% |

| Phase D: | |
|---|---|
| triéthanolamine | 3% |

| Phase E : | |
|---|---|
| éthoxydiglycol | 0,2% |
| conservateur | 0,03% |
| acacia | 5,8% |
| eau | qsp 100 |

mode opératoire : on procède comme à l'exemple 2.

Les mascaras des exemples 1 et 2 sont de couleur vive et intense. On n'a constaté aucun problème de rhéologie dans leur préparation. Ils s'étalent bien et ne tachent pas les lentilles oculaires. Les mascaras des exemples 1 et 2 sont stables à la lumière. On obtient un maquillage à la fois couvrant, saturé et brillant, les cils sont bien gainés.

### Exemple 4 : composition de mascara violet

| Phase A : | |
|---|---|
| stéarate de triéthanolamine | 10% |
| mélange de cires | 19% |

| Phase B : | |
|---|---|
| colorant polymérique bleu (*) | 3% |
| colorant polymérique rouge (**) | 1,5% |

| Phase C: | |
|---|---|
| gomme arabique | 2% |
| panthénol | 1% |
| conservateur | 0,3% |
| eau | qsp 100 |

| Phase D : | |
|---|---|
| hydroxyéthylcellulose | 1% |
| eau | 40% |

| Phase E : | |
|---|---|
| agent filmogène en dispersion aqueuse à 34% de matière active (SANCURE 2600) | 19% |

| | |
|---|---|
| (*) sulfopolyester colorant comprenant 10% de monomère colorant bleu, préparé conformément à l'exemple 4 du brevet US-4,804,719 mis en solution aqueuse à 21,6% de matière active. | |
| (**) sulfopolyester colorant préparé conformément à l'exemple 1 du brevet US-4,804,719, comprenant 10% de monomère colorant rouge, en solution aqueuse à 29% de matière active | |

Mode opératoire : Préparer D sous agitation à chaud (80°C). Laisser reposer pendant 12 heures. Homogénéiser A et chauffer à 120°C. Chauffer C+D à 80°C et ajouter B. Emulsionner A et (B+C+D). Ajouter E.

## Revendications

1. Emulsion comprenant de l'eau, un composant gras choisi parmi les huiles éventuellement volatiles et/ou les cires et un colorant polymérique, **caractérisée en ce que** le colorant polymérique est choisi parmi les polymères sulfopolyester, polyuréthanne, ou leurs mélanges.

2. Emulsion selon la revendication 1, **caractérisée par le fait que** le colorant polymérique résulte de la polymérisation de plusieurs monomères dont :
(i) au moins un résidu acide di-carboxylique portant au moins un groupement sulfonique;
(ii) au moins un résidu diol ; et
(iii) au moins un monomère colorant.

3. Emulsion selon la revendication 1 ou 2, **caractérisée en ce que** le colorant polymérique est un polymère colorant dispersible dans l'eau ayant des groupes de liaison comprenant au moins environ 20 % en moles de carbonyloxy et jusqu'à environ 80 % en moles de carbonylamido, ledit matériau contenant des groupes sulfonate hydrosolubilisants et ayant d'environ 0,01 à environ 40 % en moles, sur la base du total de tous les équivalents d'hydroxy, de carboxy ou d'amino réactifs, de colorant comprenant un ou plusieurs composés organiques thermostables ayant initialement au moins un groupe condensable, que l'on a fait réagir sur ou dans le tronc polymère.

4. Emulsion selon la revendication 3, **caractérisée en ce que** le colorant polymérique comprend :
(a) un monomère acide comprenant de 75 mole % à 84 mole % d'acide isophtalique et de 25 mole % à 16 mole % de sel de sodium de l'acide sulfo-5-isophtalique,
(b) un monomère glycol comprenant de 45 à 60 mole % de diéthylène glycol et de 55 à 40 mole % de 1,4-cyclohexanediméthanol ou d'éthylèneglycol ou leur mélange,
(c) de 0,5 à 10 mole % de monomère colorant.

5. Emulsion selon revendication 1, **caractérisée en ce que** le colorant polymérique est un polyuréthanne et résulte de la polymérisation de plusieurs monomères dont :
(i) au moins un résidu di-isocyanate ;
(ii) au moins un résidu diol ; et
(iii) au moins un monomère colorant.

6. Emulsion selon l'une quelconque des revendications précédentes, **caractérisée en ce que** le colorant polymérique comprend 5 % à 55% en poids d'au moins un monomère colorant

7. Emulsion selon la revendication 6, **caractérisée en ce que** le colorant polymérique comprend 10% à 40% en poids d'au moins un monomère colorant.

8. Emulsion selon l'une quelconque des revendications 2 à 7, **caractérisée en ce que** le monomère colorant est choisi parmi les anthraquinones, les méthines, bis-méthines, les aza-méthines, les arylidènes, les 3H-dibenzo[7,i-j] isoquinolines, les acides 2,5-diarylaminotéréphtaliques et leurs esters, les phtaloylphénothiazines, les phtaloylphénoxazines, les phtaloylacridone, les anthrapyrimidines, les anthrapyrazoles, les phtalocyanines, les quinophtalones, les indophénols, perinones, les nitroarylamines, benzodifurane, les 2 H-1-benzopyran-2-one, les quinophtalones, les perylènes, les quinacridones, les triphénodioxazines, les fluoridines, les 4-amino-1,8-naphtalimides, les thioxanthrones, les benzanthrones, les indanthrones, les indigo, thioindigo, xanthène, acridine, azine, oxazine.

9. Emulsion selon l'une quelconque des revendications précédentes, **caractérisée en ce qu'**elle comporte 0,1 à 20% de colorant polymérique.

10. Emulsion selon l'une quelconque des revendications précédentes, **caractérisée en ce qu'**elle comporte en outre au moins un composé choisi parmi le dioxyde de titane et le dioxyde de zinc.

11. Emulsion selon l'une quelconque des revendications précédentes, **caractérisée en ce qu'**elle comporte au moins une huile de silicone.

12. Emulsion selon l'une quelconque des revendications précédentes, **caractérisée en ce qu'**elle comporte au moins une cire choisie les cires d'abeilles, la cire de baleine; les cires de Camauba, de Candellila, d'Ouricoury, les cires de fibres de liège, les cires de canne à sucre et les cires du Japon; les cires de paraffine, la lanoline, les cires microcristallines, les cires de lignite, les ozokérites; les cires de polyéthylène; les cires obtenues par synthèse de Fisher et Tropsch.

13. Emulsion selon l'une des revendications précédentes, **caractérisée en ce qu'**elle comprend au moins une huile choisie parmi l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide; des huiles d'hydrocarbures telles que des huiles de paraffine, le squalane, la vaseline; des esters tels que le myristate d'isopropyle, le palmitate d'isopropyle, le stéarate de butyle, le laurate d'hexyle, l'isononanoate d'isononyle, le palmitate de 2-éthyl hexyle, le laurate de 2-hexyl décyle, le palmitate de 2-octyl décyle, le myristate de 2-octyl dodécyle, le succinate dodécyle, le triisostéarate de 2-diéthylhexyle, le malate de diisostéaryle, le lactate de 2-octyl de glycérine, le triisostéarate de glycérine, etc.; des huiles de silicone comme les polyméthylsiloxanes, les polyméthylphénylsiloxanes, des polysiloxanes modifiés par des acides gras, des polysiloxanes modifiés par des alcools gras, des polysiloxanes modifiés par des polyoxyalkylènes, des silicones fluorées, etc.; des huiles perfluorées et/ou organofluorées ;des acides gras supérieurs tels que l'acide myristique, l'acide palmitique, l'acide stéarique, l'acide béhénique, l'acide oléique, l'acide linoléique, l'acide linolénique, l'acide isostéarique, des alcools gras supérieurs tels que le cétanol, l'alcool stéarylique, l'alcool oléique.

14. Emulsion selon l'une des revendications précédentes, **caractérisée en ce qu'**elle comprend au moins une huile volatile choisie parmi les huiles de silicone telles que l'hexaméthyldisiloxane, le cyclopentadiméthylsiloxane, le cyclotétraméthylsiloxane; les huiles fluorées volatiles; les huiles isoparaffiniques.

15. Emulsion selon l'une quelconque des revendications précédentes, **caractérisée en ce qu'**elle comporte au moins un émulsionnant.

16. Emulsion selon la revendication 15, **caractérisée en ce que** l'émulsionnant est choisi parmi : les sels alcalins d'acides gras en C12-C24, les phosphatides de soja, les phospholipides, les lysophospholipides, les émulsionnants à base de silicone, les alcools gras, les esters de glycérol, les esters de sorbitan, les esters de méthylglycoside et les esters de saccharose.

17. Emulsion selon l'une quelconque des revendications précédentes, **caractérisée en ce qu'**elle se présente sous la forme d'une crème teintée transparente, d'un fond de teint, d'un rouge à lèvres, d'un eye-liner, d'un mascara, d'un produit de soin pour les lèvres, d'une poudre coulée, d'un fard à paupières ou à joues.

18. Utilisation cosmétique d'un colorant polymérique selon l'une des revendications 1 à 9 dans une émulsion pour former un film coloré sur la peau, les cils, les ongles ou les lèvres.

19. Utilisation cosmétique d'un colorant polymérique selon l'une des revendications 1 à 9 dans une émulsion qui ne colore pas la peau, les cils, les ongles ou les lèvres après le démaquillage.

20. Procédé cosmétique de maquillage des yeux, des cils, des ongles ou des lèvres, **caractérisé par le fait que** l'on applique sur les ongles, les cils, la peau ou les lèvres une émulsion selon l'une quelconque des revendications 1 à 17.

21. Utilisation cosmétique d'un colorant polymérique selon l'une des revendications 1 à 9 dans une émulsion comprenant de l'eau, un composant gras choisi parmi les huiles éventuellement volatiles et/ou les cires qui ne tâche pas et/ou ne forme pas de stries sur les lentilles oculaires.

## Patentansprüche

1. Emulsion, die Wasser, eine Fettkomponente, die unter den gegebenenfalls flüchtigen Ölen und/oder den Wachsen ausgewählt ist, und ein polymeres Farbmittel enthält, **dadurch gekennzeichnet, daß** das polymere Farbmittel unter den Sulfopolyestern, Polyurethanen oder deren Gemischen ausgewählt ist.

2. Emulsion nach Anspruch 1, **dadurch gekennzeichnet, daß** das polymere Farbmittel bei der Polymerisation mehrerer Monomere entsteht, zu denen gehören:
(i) mindestens eine Dicarbonsäure, die mindestens eine Sulfonsäuregruppe trägt,
(ii) mindestens ein Diol, und
(iii) mindestens ein monomeres Farbmittel.

3. Emulsion nach Anspruch 1 oder 2, **dadurch gekennzeichnet, daß** das polymere Farbmittel ein in Wasser dispergierbares polymeres Farbmittel ist, das Bindungsgruppen aufweist, die mindestens etwa 20 Mol-% Carbonyloxy und bis zu etwa 80 Mol-% Carbonylamido umfassen, wobei das Material wasserlöslich machende SulfonatGruppen aufweist und auf der Basis der Gesamtmenge aller reaktiven Hydroxy-, Carboxy- oder Amino-Äquivalente etwa 0,01 bis etwa 40 Mol-% Farbmittel aufweist, das ein oder mehrere wärmebeständige organische Verbindungen umfaßt, die anfänglich mindestens eine kondensierbare Gruppe enthalten, die mit dem oder innerhalb des Polymergerüsts umgesetzt werden.

4. Emulsion nach Anspruch 3, **dadurch gekennzeichnet, daß** das polymere Farbmittel enthält:
(a) ein Säuremonomer, das 75 bis 84 Mol-% Isophthalsäure und 25 bis 16 Mol-% 5-Sulfoisophthalsäure-Natriumsalz umfaßt;
(b) ein Glykolmonomer, das 45 bis 60 Mol-% Diethylenglykol und 55 bis 40 Mol-%1,4-Cyclohexandimethanol oder Ethylenglykol oder deren Gemisch umfaßt; und
(c) 0,5 bis 10 Mol-% monomeres Farbmittel.

5. Emulsion nach Anspruch 1, **dadurch gekennzeichnet, daß** das polymere Farbmittel ein Polyurethan ist, das bei der Polymerisation mehrerer Monomere entsteht, zu denen gehören:
(i) mindestens ein Diisocyanatmonomer;
(ii) mindestens ein Diolmonomer; und
(iii) mindestens ein monomeres Farbmittel.

6. Emulsion nach einem der vorhergehenden Ansprüche, **dadurch gekennzeichnet, daß** das polymere Farbmittel 5 bis 55 Gew.-% mindestens eines monomeren Farbmittels enthält.

7. Emulsion nach Anspruch 6, **dadurch gekennzeichnet, daß** das polymere Farbmittel 10 bis 40 Gew.-% mindestens eines monomeren Farbmittels enthält.

8. Emulsion nach einem der Ansprüche 2 bis 7, **dadurch gekennzeichnet, daß** das monomere Farbmittel unter Anthrachinonen, Methinen, Bismethinen, Azamethinen, Arylidenen, 3H-Dibenzo-[7,i-j]isochinolinen, 2,5-Diarylaminoterephthalsäuren und ihren Estern, Phthaloylphenothiazinen, Phthaloylphenoxazinen, Phthaloylacridonen, Anthrapyrimidinen, Anthrapyrazolen, Phthalocyaninen, Chinophtalonen, Indophenolen, Perinonen, Nitroarylaminen, Benzodifuran, 2H-1-Benzopyran-2-onen, Chinophthalonen, Perylenen, Chinacridonen, Triphenodioxazinen, Fluoridinen, 4-Amino-1,8-naphthalimiden, Thioxanthronen, Benzanthronen, Indanthronen, Indigo, Thioindigo, Xanthen, Acridin, Azin und Oxazin ausgewählt ist.

9. Emulsion nach einem der vorhergehenden Ansprüche, **dadurch gekennzeichnet, daß** sie 0,1 bis 20 Gew.-% polymeres Farbmittel enthält.

10. Emulsion nach einem der vorhergehenden Ansprüche, **dadurch gekennzeichnet, daß** sie außerdem mindestens eine Verbindung enthält, die unter Titandioxid und Zinkoxid ausgewählt ist.

11. Emulsion nach einem der vorhergehenden Ansprüche, **dadurch gekennzeichnet, daß** sie mindestens ein Siliconöl enthält.

12. Emulsion nach einem der vorhergehenden Ansprüche, **dadurch gekennzeichnet, daß** sie mindestens ein Wachs enthält, das unter Bienenwachs und Walrat; Carnaubawachs, Candelillawachs, Ouricurywachs, Korkfaserwachsen, Zuckerrohrwachsen und Japanwachsen; Paraffinwachsen, Lanolin, mikrokristallinen Wachsen, Lignitwachsen und Ozokeriten; Polyethylenwachsen; und durch Fischer-Tropsch-Synthese hergestellten Wachsen ausgewählt ist.

13. Emulsion nach einem der vorhergehenden Ansprüche, **dadurch gekennzeichnet, daß** sie mindestens ein Öl enthält, das unter Nerzöl, Schildkrötenöl, Sojaöl, Traubenkernöl, Sesamöl, Maisöl, Rapsöl, Sonnenblumenöl, Baumwollöl, Avocadoöl, Olivenöl, Ricinusöl, Jojobaöl, Erdnußöl; Kohlenwasserstoffölen, wie Paraffinölen, Squalan, Vaseline; Estern, wie Isopropylmyristat, Isopropylpalmitat, Butylstearat, Hexyllaurat, Isononylisononanoat, 2-Ethylhexylpalmitat, 2-Hexyldecyllaurat, 2-Octyldecylpalmitat, 2-Octyldodecylmyristät, Dodecylsuccinat, 2-Diethylhexyltriisostearat, Diisostearylmalat, Glycerin-2-octyllactat, Glycerintriisostearat und dergleichen; Siliconölen, wie Polymethylsiloxanen, Polymethylphenylsiloxanen, mit Fettsäuren modifizierten Polysiloxanen, mit Fettalkoholen modifizierten Polysiloxanen, mit Polyalkylenoxiden modifizierten Polysiloxanen, fluorierten Siliconen und dergleichen; perfluorierten und/oder organofluorierten Ölen; höheren Fettsäuren, wie Myristinsäure, Palmitinsäure, Stearinsäure, Behensäure, Ölsäure, Linolsäure, Linolensäure, Isostearinsäure; höheren Fettalkohlen, wie Cetanol, Stearylalkohol, Oleylalkohol, ausgewählt sind.

14. Emulsion nach einem der vorhergehenden Ansprüche, **dadurch gekennzeichnet, daß** sie mindestens ein flüchtiges Öl enthält, das unter den Siliconölen, wie Hexamethyldisiloxan, Cyclopentadimethylsiloxan, Cyclotetramethylsiloxan; fluorierten flüchtigen Ölen; und Isoparaffinen ausgewählt ist.

15. Emulsion nach einem der vorhergehenden Ansprüche, **dadurch gekennzeichnet, daß** sie mindestens einen Emulgator enthält.

16. Emulsion nach Anspruch 15, **dadurch gekennzeichnet, daß** der Emulgator ausgewählt ist unter: Alkalisalzen von C₁₂₋₁₄-Fettsäuren, Sojaphosphatiden, Phospholipiden, Lysophospholipiden, Emulgatoren auf Siliconbasis, Fettalkoholen, Glycerinestern, Sorbitanestern, Methylglycosidestern und Saccharoseestern.

17. Emulsion nach einem der vorhergehenden Ansprüche, **dadurch gekennzeichnet, daß** sie in Form einer durchsichtigen getönten Creme, eines Make-ups, eines Lippenstifts, eines Eyeliners, einer Mascara, eines Pflegeprodukts für die Lippen, eines gegossenen Pulvers, eines Lidschattens oder eines Wangenrouges vorliegt.

18. Kosmetische Verwendung eines polymeren Farbmittels nach einem der Ansprüche 1 bis 9 in einer Emulsion zur Erzeugung eines farbigen Films auf der Haut, den Wimpern, den Nägeln oder den Lippen.

19. Kosmetische Verwendung eines polymeren Farbmittels nach einem der Ansprüche 1 bis 9 in einer Emulsion, wobei die Haut, die Wimpern, die Nägel oder die Lippen nach dem Abschminken der Emulsion nicht mehr gefärbt sind.

20. Kosmetisches Verfahren zum Schminken der Augen, der Wimpern, der Nägel oder der Lippen, **dadurch gekennzeichnet, daß** auf die Nägel, die Wimpern, die Haut oder die Lippen eine Emulsion nach einem der Ansprüche 1 bis 17 aufgetragen wird.

21. Kosmetische Verwendung eines polymeren Farbmittels nach einem der Ansprüche 1 bis 9 in einer Emulsion, die Wasser und eine Fettkomponente enthält, die unter den gegebenenfalls flüchtigen Ölen und/oder den Wachsen ausgewählt ist, die keine Flecken hinterläßt und/oder keine Streifen auf Kontaktlinsen bildet.

## Claims

1. Emulsion comprising water, a fatty component chosen from optionally volatile oils and/or waxes and a polymeric dye, **characterized in that** the polymeric dye is chosen from sulphopolyester and polyurethane polymers, or mixtures thereof.

2. Emulsion according to Claim 1, **characterized in that** the polymeric dye results from the polymerization of several monomers including:
(i) at least one dicarboxylic acid residue bearing at least one sulphonic group;
(ii) at least one diol residue; and
(iii) at least one dyeing monomer.

3. Emulsion according to Claim 1 or 2, **characterized in that** the polymeric dye is a water-dispersible dyeing polymer containing bonding groups comprising at least about 20 mol% of carbonyloxy and up to about 80 mol% of carbonylamido, the said polymer containing water-solubilizing sulphonate groups and containing from about 0.01 to about 40 mol%, on the basis of the total of all of the equivalents of hydroxyl, carboxyl or amino reagents, of dye comprising one or more heat-stable organic compounds initially containing at least one condensable group, which has been reacted with or in the polymer trunk.

4. Emulsion according to Claim 3, **characterized in that** the polymeric dye comprises:
(a) an acidic monomer comprising from 75 mol% to 84 mol% of isophthalic acid and from 25 mol% to 16 mol% of sodium salt of 5-sulphoisophthalic acid,
(b) a glycol monomer comprising from 45 to 60 mol% of diethylene glycol and from 55 to 40 mol% of 1,4-cyclohexanedimethanol or ethylene glycol or a mixture thereof,
(c) from 0.5 to 10 mol% of dyeing monomer.

5. Emulsion according to Claim 1, **characterized in that** the polymeric dye is a polyurethane and results from the polymerization of several monomers including:
(i) at least one diisocyanate residue;
(ii) at least one diol residue; and
(iii) at least one dyeing monomer.

6. Emulsion according to any one of the preceding claims, **characterized in that** the polymeric dye comprises 5% to 55% by weight of at least one dyeing monomer.

7. Emulsion according to Claim 6, **characterized in that** the polymeric dye comprises 10% to 40% by weight of at least one dyeing monomer.

8. Emulsion according to any one of Claims 2 to 7, **characterized in that** the dyeing monomer is chosen from anthraquinones, methines, bis-methines, azamethines, arylidenes, 3H-dibenzo[7,i-j]isoquinolines, 2,5-diarylaminoterephthalic acids and esters thereof, phthaloylphenothiazines, phthaloylphenoxazines, phthaloylacridones, anthrapyrimidines, anthrapyrazoles, phthalocyanins, quinophthalones, indophenols, perinones, nitroarylamines, benzodifurans, 2H-1-benzopyran-2-ones, quinophthalones, perylenes, quinacridones, triphenodioxazines, fluoridines, 4-amino-1,8-naphthalimides, thioxanthrones, benzanthrones, indanthrones, indigos, thioindigos, xanthenes, acridines, azines and oxazines.

9. Emulsion according to any one of the preceding claims, **characterized in that** it comprises 0.1 to 20% of polymeric dye.

10. Emulsion according to any one of the preceding claims, **characterized in that** it also comprises at least one compound chosen from titanium dioxide and zinc dioxide.

11. Emulsion according to any one of the preceding claims, **characterized in that** it comprises at least one silicone oil.

12. Emulsion according to any one of the preceding claims, **characterized in that** it comprises at least one wax chosen from beeswax, whale wax, carnauba wax, candelilla wax, ouricury wax, cork fibre waxes, sugar cane waxes and Japan waxes, paraffin waxes, lanolin, microcrystalline waxes, lignite waxes, ozokerites, polyethylene waxes and waxes obtained by Fischer-Tropsch synthesis.

13. Emulsion according to one of the preceding claims, **characterized in that** it comprises at least one oil chosen from mink oil, turtle oil, soybean oil, grapeseed oil, sesame oil, corn oil, rapeseed oil, sunflower oil, cottonseed oil, avocado oil, olive oil, castor oil, jojoba oil, groundnut oil, hydrocarbon oils such as liquid paraffins, squalane and petroleum jelly; esters such as isopropyl myristate, isopropyl palmitate, butyl stearate, hexyl laurate, isononyl isononanoate, 2-ethylhexyl palmitate, 2-hexyldecyl laurate, 2-octyldecyl palmitate, 2-octyldodecyl myristate, dodecyl succinate, 2-diethylhexyl triisostearate, diisostearyl malate, glyceryl 2-octyl lactate, glyceryl triisostearate, etc.; silicone oils such as polymethylsiloxanes, polymethylphenylsiloxanes, polysiloxanes modified with fatty acids, polysiloxanes modified with fatty alcohols, polysiloxanes modified with polyoxyalkylenes, fluorosilicones, etc.; perfluoro oils and/or organofluorine oils; higher fatty acids such as myristic acid, palmitic acid, stearic acid, behenic acid, oleic acid, linoleic acid, linolenic acid, isostearic acid, higher fatty alcohols such as cetanol, stearyl alcohol and oleyl alcohol.

14. Emulsion according to one of the preceding claims, **characterized in that** it comprises at least one volatile oil chosen from silicone oils such as hexamethyldisiloxane, cyclopentadimethylsiloxane, cyclotetramethylsiloxane, volatile fluoro oils and isoparaffinic oils.

15. Emulsion according to any one of the preceding claims, **characterized in that** it comprises at least one emulsifier.

16. Emulsion according to Claim 15, **characterized in that** the emulsifier is chosen from: alkaline salts of C₁₂-C₂₄ fatty acids, soya phosphatides, phospholipids, lysophospholipids, silicone-based emulsifiers, fatty alcohols, glycerol esters, sorbitan esters, methylglycoside esters and sucrose esters.

17. Emulsion according to any one of the preceding claims, **characterized in that** it is in the form of a transparent tinted cream, a foundation, a lipstick, an eyeliner, a mascara, a lipcare product, a loose powder, an eyeshadow or a face powder.

18. Cosmetic use of a polymeric dye according to one of Claims 1 to 9, in an emulsion to form a coloured film on the skin, the eyelashes, the nails or the lips.

19. Cosmetic use of a polymeric dye according to one of Claims 1 to 9, in an emulsion which does not colour the skin, the eyelashes, the nails or the lips after removal of the make-up.

20. Cosmetic process for making up the eyes, the eyelashes, the nails or the lips, **characterized in that** an emulsion according to any one of Claims 1 to 17 is applied to the nails, the eyelashes, the skin or the lips.

21. Cosmetic use of a polymeric dye according to one of Claims 1 to 9, in an emulsion comprising water, a fatty component chosen from optionally volatile oils and/or waxes, which does not leave marks and/or does not form lines on ocular lenses.
